# EUROPEAN PATENT APPLICATION

(11) **EP 4 714 460 A1**
(43) Date of publication of application: **25.03.2026**
(21) Application number: 25202359.3
(22) Date of filing: 16.09.2025
(51) Int. Cl.: A61K 35/74, A61K 35/741, A61P 1/00, A61P 1/02, A61P 1/10, A61P 41/00

(54) **ESCHERICHIA COLI STRAINS FOR THE PREVENTION OF POST-COLONOSCOPY GASTROINTESTINAL DISCOMFORT**

(30) Priority: 18.09.2024 IT 202400020815
(71) Applicant: Velleja Research S.r.l., 29010 Pontenure (PC) (IT)
(72) Inventor: DI PIERRO, Francesco, 29010 PONTENURE (PC) (IT)
(74) Representative: Bianchetti & Minoja with Trevisan & Cuonzo IPS SRL

(57) **Abstract**

*E. coli* strains, administered in suitable formulations, are capable of changing the course of post-colonoscopy intestinal symptoms in subjects undergoing colonoscopies by reducing or eliminating severe discomfort with significant alterations in bowel rhythms compared to the pre-colonoscopy period.

## Description

The invention relates to the use of *Escherichia coli* strains for the treatment of discomfort resulting from colonoscopy procedures.

### Background of the invention

Colonoscopy is currently considered the method of choice for the screening and diagnosis of intestinal, inflammatory and neoplastic diseases (CA Cancer J Clin. 2021 May;71(3):209-249; Expert Rev Mol Diagn. 2022 Apr;22(4):449-460). Considered a relatively safe procedure, with an extremely low risk of incurring severe complications such as perforations and bleeding, it causes minor complications in 25% of subjects, especially bloating and abdominal pain, which can last for several days (Gastrointestinal Endosc. 2011 Oct;74(4):745-52; Gastrointest Endosc. 2007 Apr;65(4):648-56), resulting in patients losing several days of normal work and/or study activity.

Some recent evidence suggests that both the preparation for intestinal lavage and the endoscopic act itself may have an impact on the homoeostasis of the intestinal microbiota, both luminal and mucosal, with both short-term and long-term consequences (Gut. 2006 Dec;55(12):1822-3; PLoS One. 2012;7(2):e32545; Eur J Gastroenterol Hepatol. 2016 May;28(5):532-7; PLoS One. 2013 May 1;8(5):e62815; Gut. 2015 Oct;64(10):1562-8). Numerous papers have clearly shown how dysbiosis of the colonic faecal microbiota (also iatrogenic) can lead the patient to manifest gastrointestinal symptoms such as bloating and abdominal pain as well as alterations in intestinal function and motility (constipation, diarrhoea, alternate bowel motion) that can actually appear even after colonoscopy (Acta Oncol. 2019;58(sup1):S22-S28; Advances in Digestive Medicine. 2021; 8(1), 27-32).

Although probiotics have been proposed, even recently, as a potential solution to post-colonoscopy intestinal discomfort (Minerva Gastroenterol. 2024 Jun;70(2):187-196), meta-analytic analyses have clearly demonstrated their poor, or no, performance in counteracting gastrointestinal symptoms such as bloating, meteorism, flatulence, distension, pain, nausea and vomiting as well as in mitigating the appearance of irregularities of normal peristalsis (Rom J Intern Med. 2024 Jun 18) with consequences such as constipation, diarrhoea and/or alternation.

An analysis of the probiotics used to reduce the risk of intestinal discomfort after colonoscopy reveals the common use of *taxa* such as *Bacillus*, *Streptococcus*, *Lactobacillus*, *Lactococcus* and *Bifidobacterium* (Nutrients. 2023;15(5):1141; Acta Bio-Medica Atenei Parm. 2019;90(7-S):13-7; J Clin Med. 2020;9(10):3286; Dig Dis Sci. 2010;55(8):2344-51; ANZ J Surg. 2017 Sep;87(9):E65-9; Adv Biomed Res. 2018;7:94; Gastroenterol Res Pract. 2020;2020:4181748; Front Oncol. 2023;12:1078315; ANZ J Surg. 2019;89(3):234-8).

The use of *Escherichia coli (E. coli)* strains as probiotics is less common than the use of Lactobacilli and other more traditional probiotics.

The best-known *E. coli* strain used as a probiotic is *E. coli* Nissle 1917 (also known as EcN and marketed in Europe under the brand name Mutaflor^{®}), isolated during the First World War by a doctor, Alfred Nissle, who noticed that a soldier had not developed severe dysentery despite having long been exposed to conditions similar to those that had led to dysentery in his companions. The strain, isolated from that soldier's faeces (FEMS Microbiol Let. 2016 Oct;363(19): fnw212), has been used in the clinical setting for the treatment and prevention of various gastrointestinal conditions, such as ulcerative colitis, irritable bowel syndrome, constipation subtype (IBS-C) and for the prevention of gastroenteritis, especially in the paediatric setting (Microbial Ecology in Health and Disease 21.3-4 (2009): 122-158).

*E. coli* is a known opportunistic pathogen, responsible for putative urinary and intestinal infections, meningitis and septicaemia. Although the probiotic strains such as EcN are safe, they are still perceived as capable of determining a certainly greater risk for the host (Microbiol Spectr. 2020 Dec;8(4) compared to the use of lactobacilli and bifidobacteria, these being generally considered as harmless diners.

Furthermore, *Lactobacillus* and *Bifidobacterium* strains have been studied and used for decades, with a clinical database and much broader clinical use experience than the few *E. coli* probiotics.

Specifically, in addition to the aforementioned EcN, the following *E. coli* strains are considered as probiotics:
1) *E. coli* G1/2, G3/10, G4/9, G5, G6/7, G8 colibactin-positive (DSM 17252), isolated from a healthy individual in 1954 and currently on the market in a mixture in specific ratios (Symbioflor2^{®}) for the therapy of IBS, (Z Gastroenterol. 2009 Feb;47(2):209-14);
2) *E. coli* A0 34/86 (ATCC 25922) colibactin-positive, isolated from pig faeces in 1968 and used in the prevention of infections and atopy of the infant (Colinfant New Born^{®}).

Lactobacilli and bifidobacteria produce lactic acid and acetic acid, respectively. They thus reduce the intestinal pH and inhibit the growth of pathogens. Instead, *E. coli* Nissle 1917 acts through different mechanisms, such as the strengthening of the intestinal barrier and direct competition with entero-pathogens, especially at the mucosal level, in the same ecological niche.

### Description of the Invention

It has now been found that *E. coli* strains are useful as probiotics in the treatment of intestinal discomfort following colonoscopy procedures. In particular, the use of *E. coli* strains proved to radically change the course of post-colonoscopy intestinal symptoms in subjects who in previous colonoscopies had reported severe discomfort with significant alterations in the rhythms of the bowel compared to the pre-colonoscopy period.

The term discomfort refers to gastrointestinal symptoms comprising or consisting of nausea, bloating, distension, flatulence and pain.

In particular, pks-negative *E. coli* strains are preferred, that is, lacking the so-called pks island, a genomic area that encodes colibactin (mutagen) and colibactin resistance. To date, no probiotic *E. coli* strain has been found to be pks-negative. Only one pks-negative *E. coli* strain isolated from lactating rats (*E. coli* CEC15) is known but is not usable as a probiotic as it is characterized by intrinsic virulence and on a genomic basis). It was therefore never marketed for safety reasons.

Particularly preferred is the pks-negative *E.coli* strain 5C or ECS24, deposited in the Gent collection in Belgium under accession number LMS- S 33222, isolated in 2022 from the faeces of a healthy infant. Said strain has the genome reported in Microbiology Resources Announcements published on 16.09.2024 (://journals.asm.org/doi/epub/10.1128/mra.00580-24).

The genome of LMS-S 33222 was deposited with NCBI under BioProject accession numbers 62 PRJNA1114436 (https://www.ncbi.nlm.nih.gov/bioproject/?term=PRJNA1114436 and with the corresponding GenBank accession number JBDPNK000000000.1 (64 (https://www.ncbi.nlm.nih.gov/nuccore/JBDPNK000000000).

Also preferred are strains with genomes having a degree of homology with respect to said deposited sequences of 85%, preferably 90%, more preferably 95% or higher. The invention also relates to compositions containing said strains.

Other *E. coli* strains that can be used in accordance with the invention are Nissle 1917 (DSM 6601) and A034/86 (ATCC 25922).

The strains, in live, inactivated or dead form, can be used in combination with other probiotics, prebiotics, vitamins, plant extracts, antioxidants, mineral salts or other active ingredients with complementary or otherwise useful activity. Abiotic supernatants, possibly in lyophilized, dried or liquid form, obtained from the cultivation of the strains, in particular LMS-S 33222 strain or homologous, can also be used.

The strains for the use of the invention may be administered orally or locally, for example by colonic irrigation, in dosages typically comprised between 1 x 10⁶ CFU and 1 x 10¹². Examples of suitable formulations are provided below.

### Formulation examples

### 1) Enteric-coated capsule

| *Ingredient* | *Dose* |
|---|---|
| *E coli* LMG S-33222 | > 1 x 10⁶ CFU |
| Mg stearate | 100 mg |
| Silicon Dioxide | 100 mg |

### 2) Oral sachets

| *Ingredient* | *Dose* |
|---|---|
| *E coli* LMG S-33222 heat-killed | > 1 x 10⁶ CFU (dose calculated before inactivation) |
| Abiotic *E. coli* supernatant | > 50 mg (lyophilized) |
| Maltodextrins | 100 mg |
| Rice starch | 100 mg |
| FOS | 1500mg |

### 3) Preparation for colonic inoculation

| *Ingredient* | *Dose* |
|---|---|
| *E coli* LMG S-33222 | > 1 x 10⁹ CFU |
| Isotonic Solution | 50 ml |
| Ascorbate | 100 mg |

### Clinical trial

10 individuals who after previous colonoscopies had reported severe intestinal symptoms and changes in bowel regularity were administered, immediately after colonoscopy and in the following 4 days, with one dose every 12 hours of LMG S-33222 strain (formulated at no less than 1 billion CFU per dose in enteric-protected capsules). The colon preparation used corresponded to 1 litre of polyethylene glycol plus ascorbate (Plenvu^{®}; Norgine, Amsterdam, Netherlands). Each treatment was administered as a divided dose regimen. The first dose was administered the evening before the endoscopic examination, at 8.00 pm. The second dose was administered on the morning of the examination at 6.00 am, i.e. within 5 hours of the start of the colonoscopy. In all cases the colon preparation used was identical to those used in the previous colonoscopies. All patients reported complete intake of wash solutions. During and after bowel preparation, solid food was not allowed. Clear liquids could be taken up to 2 hours prior to the procedure.

Conscious sedation and mild analgesia were used for colonoscopy according to the preferences of the digestive endoscopy department involved in the trial. The colonoscopy was performed using standard optical high-definition endoscopes. Examination was considered complete if the caecum and the first portion of the ileum were visualized. Although no lesions (even benign) were observed in any patient, the trial was performed so that all possible lesions detected were measured with open biopsy forceps and annotated based on size, morphology, and location, and so that advanced adenomas were defined as adenomas ≥10 mm in diameter, with villous component, with high-grade or cancerous dysplasia.

With regard to the evaluation of the symptoms and the appearance of any side effects, the evaluation lasted 15 days and was measured by visual analogue scale (VAS: 0-10) with 0=no symptoms and 10= intolerable symptoms. The gastrointestinal symptoms measured included nausea, bloating, distension, flatulence and pain. For the detection of the bowel movements, the Bristol scale (1-7) was used to classify the faeces according to their shape and consistency.

The table below shows the results obtained (expressed as average ± standard deviation).

**Table 1. Evolution of symptoms in patients (N=10) according to a VAS (0-10) after colonoscopy**

| Day | Swelling | Flatulence | NauseaPain | BSS | | Antibiotics |
|---|---|---|---|---|---|---|
| 1 | 5±2.5 | 5±1 | 2±1 | 2±1.5 | ND | No |
| 2 | 2±0.5 | 3±1 | 0±0.5 | 1±0.5 | ND | No |
| 3 | 2±0.5 | 2±1 | 0±0.5 | 1±0.5 | 5±1.5 | No |
| 4 | 1±0.5 | 1±1.5 | 0 | 1±0.5 | 4±1 | No |
| 5 | 2±1 | 2±1.5 | 0 | 1±1 | 4±0.5 | No |
| 10 | 3±1 | 2±1 | 0 | 0±0.5 | 4±0.5 | No |
| 15 | 2±0.5 | 1±1 | 0 | 1±0.5 | 3±1 | No |

| | | | | | | |
|---|---|---|---|---|---|---|
| VAS: visual analogue scale; BSS: Bristol stool score; ND: not detected (no spontaneous evacuation occurred). | | | | | | |

## Claims

1. *Escherichia coli* strains for use in the treatment of discomfort following colonoscopy procedures.

2. Strains for use according to claim 1 which are pks-negative.

3. A strain for use according to claim 1 or 2 which has the same or homologous genome as strain LMS-S 33222.

4. A strain for use according to any one of claims 1 to 3 wherein discomfort comprises nausea, bloating, distension, flatulence and pain.

5. Probiotic compositions comprising an *Escherichia coli* strain having the same or homologous genome as strain LMS-S 33222 or an abiotic supernatant thereof.

6. Compositions according to claim 5 further comprising other probiotics, prebiotics, vitamins, plant extracts, antioxidants, mineral salts.
